(19) **Europäisches Patentamt**
**European Patent Office**
**Office européen des brevets**

(11) **EP 3 277 371 B1**

(12) **EUROPEAN PATENT SPECIFICATION**

(45) Date of publication and mention
of the grant of the patent:
**26.08.2020 Bulletin 2020/35**

(21) Application number: **16719931.4**

(22) Date of filing: **30.03.2016**

(51) Int Cl.:
*A61N 1/378* (2006.01)    *A61N 1/372* (2006.01)
*A61N 1/36* (2006.01)    *H02J 5/00* (2016.01)
*H02J 7/02* (2016.01)    *H01F 38/14* (2006.01)
*A61F 2/915* (2013.01)    *A61N 1/05* (2006.01)
*A61N 1/362* (2006.01)    *A61B 5/00* (2006.01)

(86) International application number:
**PCT/IL2016/050338**

(87) International publication number:
**WO 2016/157183 (06.10.2016 Gazette 2016/40)**

(54) **ANTENNA FOR USE WITH AN INTRAVASCULAR DEVICE**

ANTENNE ZUR VERWENDUNG MIT EINER INTRAVASKULÄREN VORRICHTUNG

ANTENNE POUR UTILISATION AVEC UN DISPOSITIF INTRAVASCULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2015  US 201562140141 P**

(43) Date of publication of application:
**07.02.2018 Bulletin 2018/06**

(60) Divisional application:
**20179190.2**

(73) Proprietor: **Enopace Biomedical Ltd.**
**38900 Caesarea (IL)**

(72) Inventors:
• **DAGAN, Amir**
**19230 Kibbutz Megiddo (IL)**
• **GINDIN, Igor**
**3269112 Haifa (IL)**

• **FICHMAN, Mark**
**3706207 Pardes Hana Karkur (IL)**
• **KATZ, Yoav**
**3050000 Binyamina (IL)**
• **LIPPERMAN, Fabian**
**44650 Kfar Saba (IL)**
• **PELEG, Nadav**
**4281000 Zur Moshe (IL)**
• **ARIAV, Gal**
**3780800 Givat Ada (IL)**

(74) Representative: **Evens, Paul Jonathan et al**
**Maguire Boss**
**24 East Street**
**St. Ives, Cambridgeshire PE27 5PD (GB)**

(56) References cited:
**EP-A1- 1 703 638    US-A1- 2012 239 107
US-A1- 2013 310 629    US-A1- 2014 025 140
US-A1- 2014 180 391    US-A1- 2014 197 786
US-A1- 2014 265 620**

**Description**

**FIELD OF THE INVENTION**

Some applications of the present invention generally relate to medical apparatus. Specifically, some applications of the present invention relate to stent-based electrodes for placement in a blood vessel.

**BACKGROUND**

Heart failure is a condition in which a problem with the structure or function of the heart impairs its ability to supply sufficient blood flow to meet the body's needs. The condition impairs quality of life and is a leading cause of hospitalizations and mortality in the Western world. Treatment of heart failure is typically aimed at removal of precipitating causes, prevention of deterioration in cardiac function, and control of congestive state.

Hypertension, or chronic high blood pressure, is an extremely prevalent medical condition, which can lead to strokes, heart attacks, and heart failure. There are a variety of treatments that are available for treating hypertension, including lifestyle changes and medication.

**SUMMARY OF THE INVENTION**

The present invention is defined by the appended claims.

Aspects, applications, embodiments or examples of the present disclosure that do not fall within the scope of the appended claims do not form part of the present invention. Methods described hereinafter are also not part of the present invention.

For some applications of the present disclosure a stent is inserted into a subject's aorta. Typically, the stent has one or more electrodes and a first antenna coupled thereto. A second antenna is placed on the subject, such that the second antenna extends at least from above a left clavicle of the subject to below a jugular notch of the subject and from below the subject's jugular notch to above a right clavicle of the subject. The second antenna transmits an electrical signal to the first antenna via inductive coupling.

For some applications, the stent is placed within a lumen e.g., a lumen of a blood vessel of a subject, such as the subject's aorta. Typically, the stent defines a stent body, a plurality of antenna posts that protrude from a distal end of the stent body, a plurality of electrode posts that protrude from the distal end of the stent body, and one or more coupling elements for coupling a control capsule to the inner surface of the stent body. Further typically, the first antenna is coupled to the stent by being sutured to the antenna posts that protrude from the stent body, a plurality of coiled electrodes are coupled to the stent by being placed upon respective electrode posts, and a control capsule is coupled to stent via the coupling elements. For some applications, a control unit and the second antenna (i.e., the transmitter) are disposed outside the subject's body. The control unit transmits a signal and/or power toward the stent, via the transmitter. The antenna on the stent receives the transmitted signal and/or power, and the control capsule drives the electrodes to drive a current into the blood vessel, in response to the antenna receiving the signal and/or power.

Typically the stent is placed inside the subject's aorta such that the distal end of the stent is in the vicinity of the subject's aortic arch. For some applications, the stent is placed such that the electrodes are disposed between the bifurcation of the aorta with the left subclavian artery and the bifurcation of the aorta with the fifth intercostal artery. For some applications, the control capsule drives the electrodes to drive a current into the subject's aorta, e.g., in order to treat the subject for a condition such as congestive heart failure, diastolic heart failure, and/or hypertension, e.g., as described in US 13/210,778 to Dagan (published as US 2012/0035679), US 12/957,799 to Gross (published as US 2011/0137370), and/or US 12/792,227 to Gross (published as US 2010/0305392).

It is noted that in the context of the present disclosure, the terms "proximal" and "distal" are to be understood to be with respect to an access point of the stent into the subject's body. Thus, the distal end of the stent is the end of the stent that is further from the access point, and the proximal end of the stent is the end of the stent that is closest to the access point. For applications in which the stent is placed inside the subject's aorta, the term "distal" typically means the portion of the stent or the aorta that is closer to the subject's left ventricle, and the term "proximal" means the portion of the stent or the aorta that is further from the subject's left ventricle.

There is therefore provided, in accordance with the present invention, apparatus as defined in claim 1. Preferred embodiments are defined by the dependent claims.

In some applications, at least a portion of the stent is configured to be placed in a descending aorta of the subject.

In some applications, at least a portion of the stent is configured to be placed in an aortic arch of the subject.

In some applications, the stent is configured to be placed in the aorta such that the electrodes are placed in contact with a site disposed between a bifurcation of the aorta with a left subclavian artery and a bifurcation of the aorta with a fifth intercostal artery.

In some applications, the stent and the second antenna are configured such that when the stent is placed in the aorta, and the second antenna is placed on the subject such that the second antenna extends at least from above the subject's left clavicle to below the subject's jugular notch and from below the subject's jugular notch to above the subject's right clavicle, a coupling coefficient between the first and second antennas is greater than 0.004, the coupling coefficient being defined as:

$$\text{coupling coefficient} = (V_1/V_2) * \sqrt{(L_2/L_1)}$$

where $V_1$ and $V_2$ are voltage gains of the first and second antennas, respectively, and $L_1$ and $L_2$ are inductances of the first and second antennas, respectively.

In some applications, the second antenna is configured to be implanted subcutaneously.

In some applications, the second antenna is configured not to circumscribe a neck of the subject.

In some applications, the second antenna is configured to circumscribe a neck of the subject.

In some applications, the second antenna includes at least one turn of wire configured to form a complete loop that extends from below the subject's jugular notch to behind a vertebra of the subject that is between C1 and T3 vertebrae.

In some applications, the stent and the second antenna are configured such that when the stent is placed in the aorta, and the second antenna is placed on the subject such that the second antenna extends at least from above the subject's left clavicle to below the subject's jugular notch and from below the subject's jugular notch to above the subject's right clavicle, an effective sagittal angle between the first and second antennas is between 20 and 70 degrees.

In some applications, the stent and the second antenna are configured such that when the stent is placed in the aorta, and the second antenna is placed on the subject such that the second antenna extends at least from above the subject's left clavicle to below the subject's jugular notch and from below the subject's jugular notch to above the subject's right clavicle, the effective sagittal angle between the first and second antennas is between 30 and 60 degrees.

In some applications, the second antenna includes a plurality of turns of wire, and a plurality of capacitors, and each of the turns of wire is coupled to a respective one of the capacitors.

In some applications, the capacitors are configured to prevent the second antenna from becoming detuned as a result of becoming misshapen.

The apparatus of the present invention finds application in an exemplary method including:

inserting into an aorta of a subject a stent having one or more electrodes, and a first antenna coupled thereto; and placing a second antenna on the subject, such that the second antenna extends at least from above a left clavicle of the subject to below a jugular notch of the subject and from below the subject's jugular notch to above a right clavicle of the subject,
the second antenna being configured to transmit an electrical signal to the first antenna via inductive coupling.

In some applications, the method further includes operating the second antenna to transmit power to the first antenna, such that a current is driven a current into the subject's aorta via the electrodes, using the received power.

In some applications, inserting the stent into the subject's aorta includes inserting at least a portion of the stent into a descending aorta of the subject.

In some applications, inserting the stent into the subject's aorta includes inserting at least a portion of the stent into an aortic arch of the subject.

In some applications, inserting the stent into the subject's aorta includes inserting the stent such that the electrodes are placed in contact with a site disposed between a bifurcation of the aorta with a left subclavian artery and a bifurcation of the aorta with a fifth intercostal artery.

In some applications, placing the second antenna on the subject such that the second antenna extends at least from above the subject's left clavicle to below the subject's jugular notch and from below the subject's jugular notch to above the subject's right clavicle includes placing the second antenna such that a coupling coefficient between the first and second antennas is greater than 0.004, the coupling coefficient being defined as:

$$\text{coupling coefficient} = (V_1/V_2) * \sqrt{(L_2/L_1)},$$

where $V_1$ and $V_2$ are voltage gains of the first and second antennas, respectively, and $L_1$ and $L_2$ are inductances of the first and second antennas, respectively.

In some applications, placing the second antenna on the subject includes subcutaneously implanting the second antenna.

In some applications, placing the second antenna on the subject includes supporting the second antenna on the subject such that the second antenna extends at least from above the subject's left clavicle to below the subject's jugular notch and from below the subject's jugular notch to above the subject's right clavicle using a support selected from the group consisting of: an undershirt, a bra-like housing, silicone configured to stick to skin of the subject, and string.

In some applications, placing the second antenna on the subject includes placing the second antenna on the subject such that the second antenna does not circumscribe a neck of the subject.

In some applications, placing the second antenna on the subject includes placing the second antenna on the subject such that the second antenna circumscribes a neck of the subject.

In some applications, placing the second antenna on the subject includes placing the second antenna on the subject such that at least one turn of wire of the second antenna forms a complete loop that extends from below the subject's jugular notch to behind a vertebra of the subject that is between C1 and T3 vertebrae.

In some applications, placing the second antenna on the subject such that the second antenna extends at least from above the subject's left clavicle to below the subject's jugular notch and from below the subject's jugular notch to above the subject's right clavicle includes placing the second antenna such that when the stent is inside the aorta an effective sagittal angle between the first and second antennas is between 20 and 70 degrees.

In some applications, placing the second antenna on the subject such that the second antenna extends at least from above the subject's left clavicle to below the subject's jugular notch and from below the subject's jugular notch to above the subject's right clavicle includes placing the second antenna such that when the stent is inside the aorta the effective sagittal angle between the first and second antennas is between 30 and 60 degrees.

In some applications, placing the second antenna on the subject includes placing on the subject a second antenna that includes a plurality of turns of wire, and a plurality of capacitors, each of the turns of wire being coupled to a respective one of the capacitors.

In some applications, placing on the subject a second antenna that includes a plurality of turns of wire, and a plurality of capacitors, each of the turns of wire being coupled to a respective one of the capacitors includes preventing the second antenna from becoming detuned as a result of becoming misshapen.

The present invention will be more fully understood from the following detailed description of embodiments thereof, taken together with the drawings, in which:

## BRIEF DESCRIPTION OF THE DRAWINGS

Fig. 1 is a schematic illustration of a stent having electrodes and an antenna disposed thereon, the stent having been placed inside a subject's aorta;

Figs. 2A-B are schematic three-dimensional illustrations showing respective views of a self-expandable stent in its expanded configuration;

Figs. 3A-C are schematic illustrations showing the structure of a stent for placing inside a blood vessel;

Figs. 4A-C are schematic illustrations of respective views of an antenna disposed on a subject's upper body;

Figs. 5A-C are schematic illustrations of respective views of the antenna shown in Figs. 4A-C;

Fig. 6 is a schematic illustration of another antenna placed on a subject's upper body;

Fig. 7 is a schematic illustration of the antenna shown in Fig. 6;

Fig. 8 is a schematic illustration of an antenna for placing on a subject's upper body; and

Fig. 9 is a graph that schematically illustrates stimulation and power-transmission cycles of an intravascular device and an external transmitter.

## DETAILED DESCRIPTION OF EMBODIMENTS

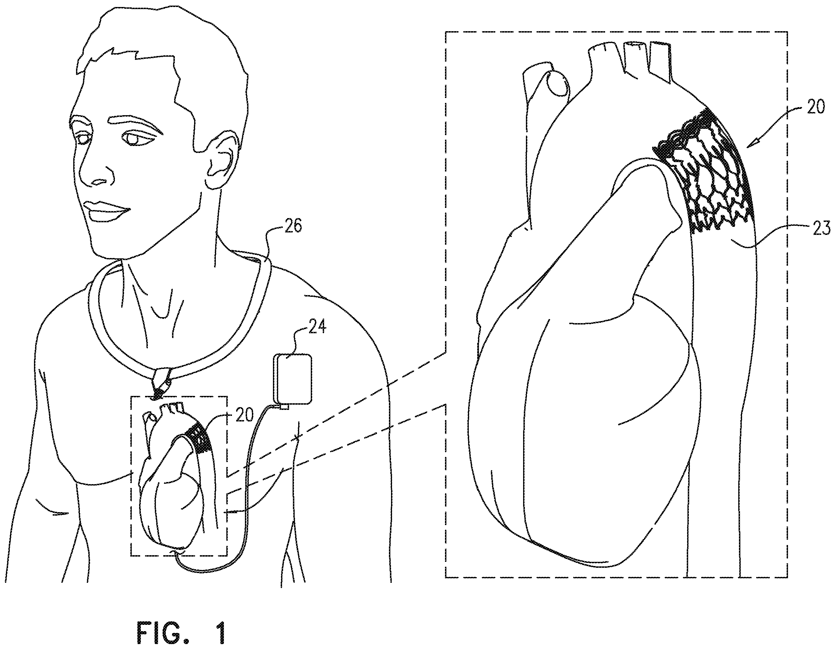

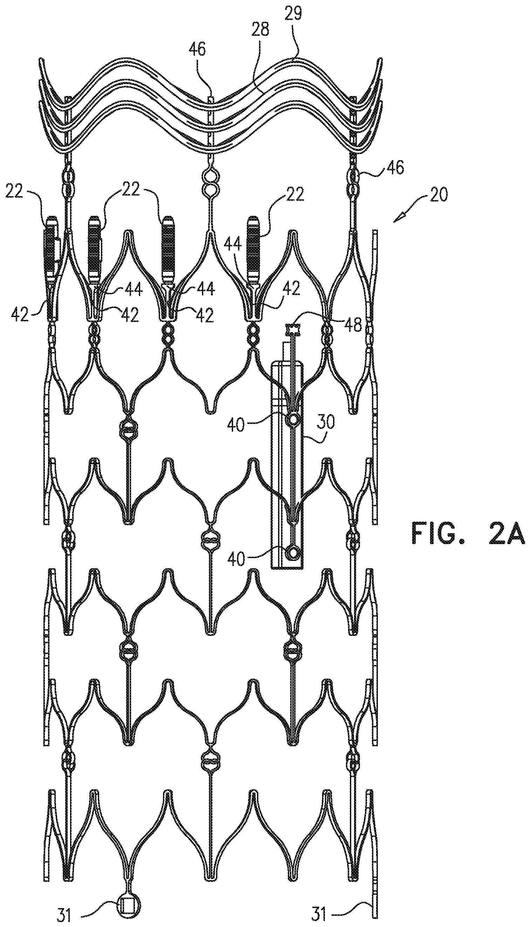

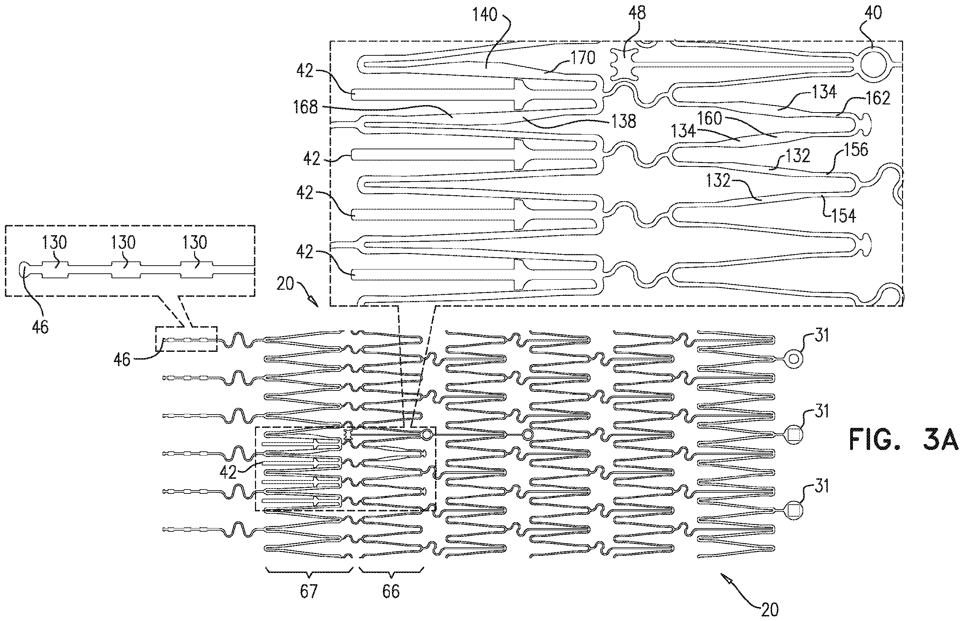

Reference is now made to Fig. 1, which is a schematic illustration of a self-expandable stent 20 placed inside a lumen, at least one electrode 22 (Figs. 2A-B), and typically, a plurality of electrodes, being disposed on the stent, in accordance with some applications of the present disclosure. For some applications, stent 20 is placed inside a subject's blood vessel, stent 20 typically being placed inside the subject's aorta 23, as shown. Reference is also made to Figs. 2A-B, which are schematic illustrations of the stent in its expanded configuration, and in the absence of the subject's anatomy, in accordance with some applications of the present disclosure. It is noted that the stent as depicted in Fig. 1 is illustrative, and that the appearance of the stent is typically as shown and described with reference to Figs. 2A-B, and Figs. 3A-C.

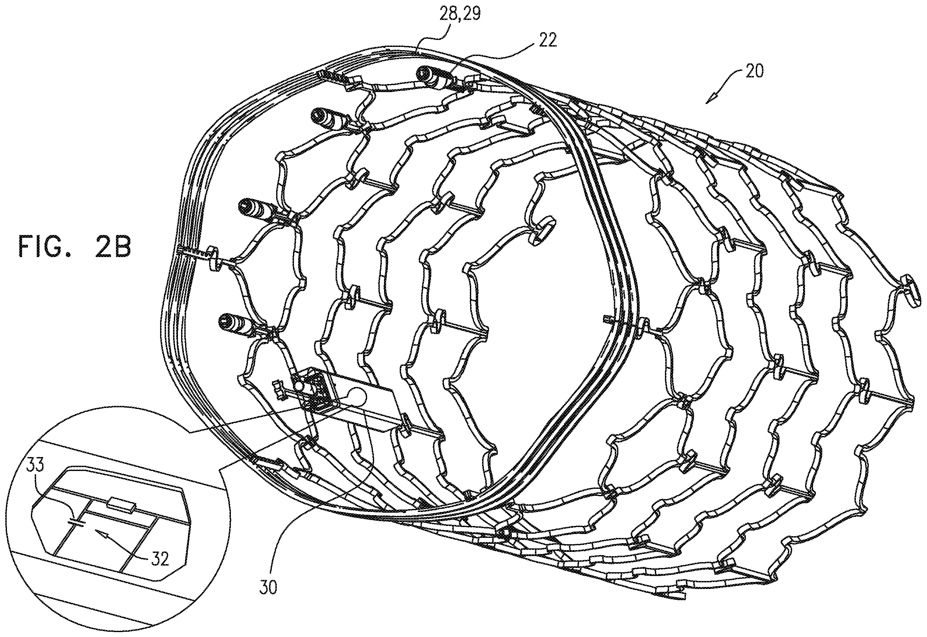

Typically, a control unit 24 and a transmitting antenna (i.e., a transmitter) 26 are disposed outside the subject's body, as shown in Fig. 1. The control unit typically comprises circuitry, and/or includes a computer processor (i.e., a structural computer processing unit that includes hardware elements). For some applications (not shown), the control unit and/or the transmitter are implanted (e.g., subcutaneously implanted) inside the subject's body. Typically, an antenna 28 and a control capsule 30 (Figs. 2A-B) are coupled to stent 20. Control capsule 30 houses control circuitry 32 (Fig. 2B), which, typically, includes a capacitor 33. Control unit 24 transmits a signal and/or power toward stent 20, via transmitter 26. Antenna 28 receives the transmitted signal and/or power, and control circuitry 32 of control capsule 30 drives the electrodes to drive a current into the blood vessel, in response to the antenna receiving the signal. For some applications, control circuitry 32 of control capsule 30 transmits data to control unit 24 by transmitting a signal from antenna 28 toward antenna 26. Thus, for some applications, antenna 28 may also act as a transmitter, and antenna 26 may also act as a receiver. Typically, antenna 26 and antenna 28 communicate with one another via electromagnetic inductive coupling. For some applications, control unit 24 is programmable using a computer. For example, a user (such as a physician) may use a computer (not shown) to program control unit 24, using a standard communication protocol, such as Bluetooth®, to facilitate communication between the computer and control unit 24.

Typically, electrodes 22 are placed in contact with an aortic site, which is as described in US 13/210,778 to Dagan (issued as US 8,626,290), US 12/957,799 to Gross (issued as US 8,626,299), and/or US 12/792,227 to Gross (published as US 2010/0305392) The aortic site is typically between the bifurcation of the aorta with the left subclavian artery and the bifurcation of the aorta with the fifth intercostal artery. Further typically, the aortic site is between the bifurcation of the aorta with the left subclavian artery and the bifurcation of the aorta with the fourth intercostal artery, e.g., between the bifurcation of the aorta with the left subclavian artery and the bifurcation of the aorta with the first intercostal artery. For some applications, the aortic site is between the bifurcations of the aorta with the first and fifth intercostal arteries. For some applications, at least a portion of the stent is placed inside the descending aorta, and/or at least a portion of the stent is placed in the aortic arch.

Typically, a current is driven into the subject's aorta, e.g., in order to treat the subject for a condition such as congestive heart failure, diastolic heart failure, and/or hypertension, e.g., as described in US 13/210,778 to Dagan (issued as US 8,626,290), US 12/957,799 to Gross (issued as US 8,626,299), and/or US 12/792,227 to Gross (published as US 2010/0305392) For some applications, stent 20 is cut from a nitinol tube (or a tube made from a different material, such as stainless steel) having a wall thickness of more than 0.2 mm (e.g., more than 0.4 mm), and/or less than 0.7 mm (e.g., less than 0.6 mm). For some applications, the length of the stent is more than 25 mm (e.g., more than 30 mm), and/or less than 100 mm (e.g., less than 40 mm). The stent is shape set to a desired expanded configuration of the stent, using techniques that are known in the art. For some applications, the stent is shape set such that in its expanded configuration (i.e., in the absence of any forces acting on the stent), the stent has a maximum outer diameter of more than 10 mm (e.g., more than 15 mm), and/or less than 60 mm (e.g., less than 50 mm). The stent is typically configured such that, upon being deployed in its expanded configuration inside the subject's aorta, the stent anchors itself within the aorta by at least the ends of the stent body (and, typically, the entire stent body) expanding such as to contact the inner wall of the aorta. Furthermore, the stent is typically configured such that, upon being deployed in its expanded configuration inside the subject's aorta, the stent maintains electrodes 22 in contact with the aortic site, and the stent maintains antenna 28 in an open configuration, as described in further detail hereinbelow.

Stent 20 is typically configured to be placed inside the blood vessel (e.g., the aorta) percutaneously (e.g., transfemorally) using a delivery system, e.g., using a 12 Fr - 20 Fr catheter (e.g., a 16 Fr catheter). In order to facilitate the percutaneous placement of the stent into the blood vessel (e.g., the aorta), using the catheter, the stent is crimped. Typically, the stent has a crimped profile of less than 20 Fr (e.g., 18 Fr or less), and/or more than 8 Fr (e.g., 10 Fr or more). Typically, stent 20 defines coupling elements 31 at a proximal end of the stent. For some applications, the coupling elements are disposed at a location along the length of the stent other than the proximal end of the stent. During insertion of the stent via the catheter, the delivery system holds the stent in place with respect to the catheter using the coupling elements. In order to place the stent inside the blood vessel at a deployment location, the catheter is retracted at the deployment location, such that the stent is released from the catheter. The stent becomes anchored to the blood vessel via radial expansion of the stent against the inner wall of the blood vessel. Subsequently, the coupling elements are decoupled from the delivery system, and the catheter is withdrawn from the blood vessel.

For some applications, upon being placed inside the blood vessel, the stent is partially deployed by retracting the catheter with respect to the stent, such that (a) electrodes 22 contact the wall of the blood vessel at a given location within the blood vessel, and (b) a proximal portion of the stent is disposed inside the catheter, such that the stent may be retrieved into the catheter. For some applications, the response of the subject to electrical stimulation of the blood vessel at the current location of the electrodes within the blood vessel is determined. In response thereto, the stent is (a) fully deployed at the current location of the stent, (b) retrieved into the catheter and redeployed at a different location within the blood vessel, or (c) retrieved into the catheter and removed from the subject's body (e.g., if the subject does not respond in a suitable manner to electrical stimulation of the blood vessel at any location at which the stent is deployed). Alternatively or additionally, prior to stent 20 being placed inside the blood vessel (e.g., inside the aorta), a mapping device is placed inside the blood vessel, the mapping device including stimulation electrodes. The subject's blood vessel is electrically stimulated at a plurality of stimulation sites using the stimulation electrodes of the mapping device, and the subject's response to electrical simulation at respective sites within the blood vessel is monitored. Subsequently, the mapping device is retrieved from the blood vessel, and stent 20 is placed inside the blood vessel. The location at which to deploy stent 20 within the blood vessel is determined, in response to the monitoring of the subject's responses to the stimulation at the respective sites using the mapping device.

Typically, the compliance of stent 20 is such that pulsation of the blood vessel is substantially maintained upon the stent being deployed inside the blood vessel. Further typically, the stent and components coupled thereto (such as control capsule 30) are shaped such as to substantially maintain blood flow through the blood vessel upon deployment of the stent inside the blood vessel.

As shown in Fig. 2A, stent 20 typically defines one or more coupling elements 40 for facilitating coupling of control capsule 30 to the stent. For some applications, as shown, the stent defines rings that define holes. The control capsule is coupled to the stent by inserting protrusions that protrude from the back of the control capsule into the rings. As shown, the control capsule is typically configured to be coupled to the stent such that the control capsule is disposed on the inner surface of the stent. For some applications, a length of the control capsule is more than 10 mm, less than 30 mm (e.g., less than 25 mm), and/or 10-30 mm (e.g., 10-25 mm). Typically, the width and depth of the control capsule are each greater than 1 mm (e.g., greater than 2 mm), less than 5 mm (e.g., less than 3 mm), and/or 1-5 mm (e.g., 2-3 mm).

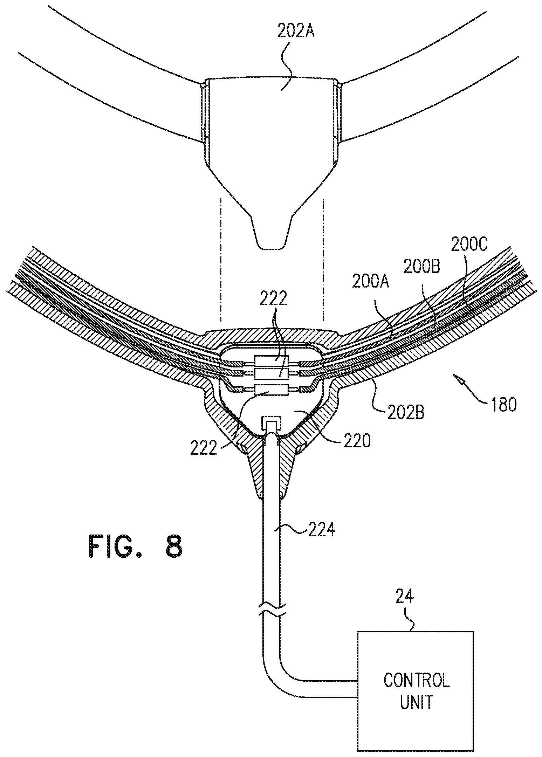

Typically, at least one electrode 22 is configured to be coupled to stent 20. For some applications, electrode 22 is coiled and is coupled to stent 20 by being placed upon an electrode post 42 that protrudes from the body of stent 20. The electrode is typically disposed on an electrode construction that is configured such that, when the electrode construction is placed on the electrode post, electrode 22 is electrically isolated from the antenna and from the stent body, for example as described with reference to Figs. 8A-B of US 2014/0180391 For some applications, electrode post 42 is shaped to define protrusions 44, such as to prevent the electrode construction from sliding toward the stent body, when the electrode construction is coupled to the electrode post. Typically, at least 2 electrodes (e.g., at least 3 electrodes), and/or less than 12 electrodes (e.g., less than 6 electrodes) are coupled to stent 20, respective electrodes being placed upon respective electrode posts that protrude from the stent body. For example, 3-5 electrodes (e.g., 4 electrodes) may be coupled to stent 20, respective electrodes being placed upon respective electrode posts that protrude from the stent body.

Typically, antenna 28 is made of a metal wire, e.g., a gold wire. In order for transmitter 26 to communicate with antenna 28 via inductive coupling, it is typically desirable that the antenna become fully expanded inside the blood vessel, such that the antenna is in contact with the inner wall of the blood vessel. For some applications, in order to facilitate expansion of the antenna inside the subject's blood vessel, nitinol wire 29 is coupled to the gold wire, and the nitinol wire is shape set in a desired expanded configuration of the antenna. The distal end of the delivery catheter that is used to deliver stent 20 is retracted at the deployment location of the stent, as described hereinabove. The retraction of the delivery catheter causes the nitinol wire to self-expand inside the subject's blood vessel, and due to the coupling of the nitinol wire to the gold wire, the nitinol wire causes the antenna to expand into the desired expanded configuration (e.g., such that the antenna is in contact with the inner wall of the blood vessel). Typically, the antenna includes a plurality of turns of the gold wire. For example, the antenna may include more than 2 turns, and/or less than 12 turns, e.g. 2-12 turns or 2-6 turns. For some applications, the antenna includes 6 turns of the gold wire, the six turns of wire being separated into 3 levels that are separated from another, as shown. For some applications, the antenna wires are shaped in a waved configuration, as shown.

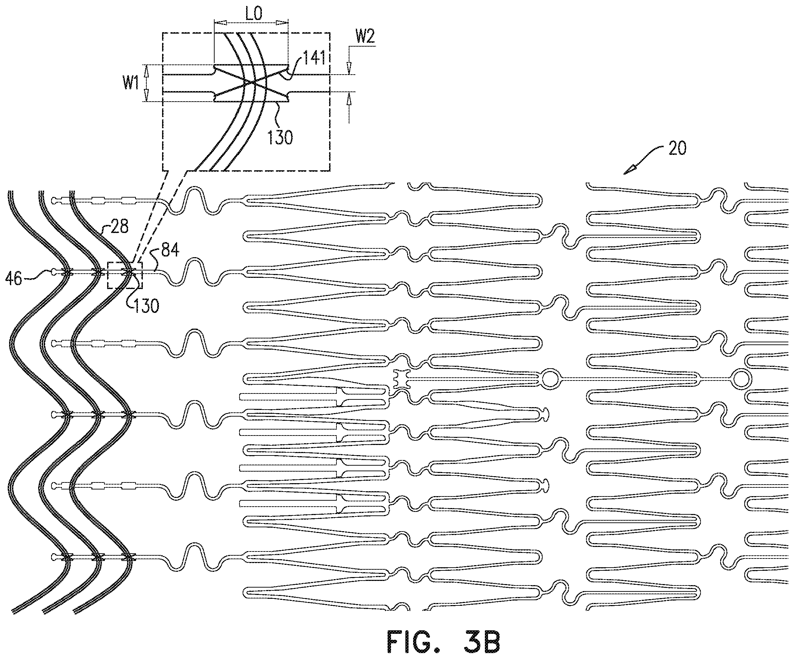

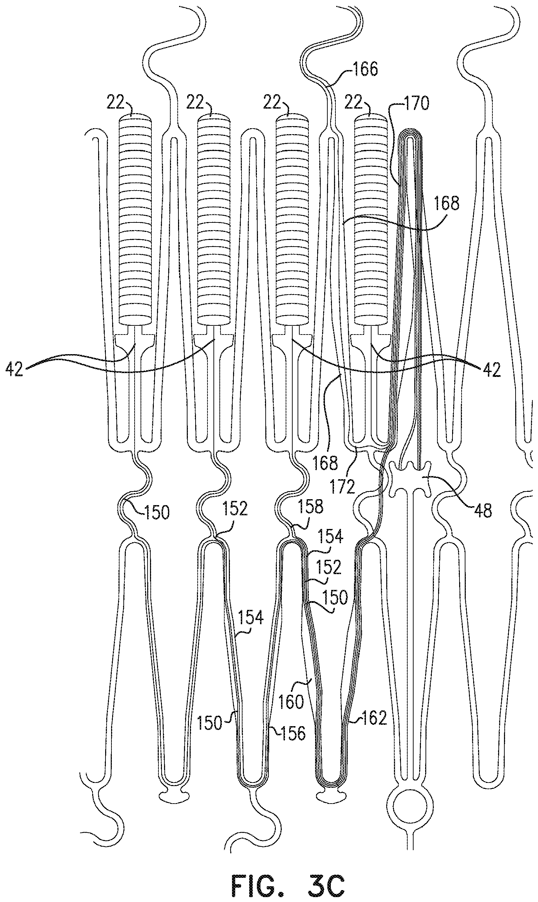

Antenna 28 and nitinol wire 29 are typically coupled to stent 20 by being sutured to antenna posts 46, which protrude from the stent body of stent 20 separately from electrode posts 42. As described hereinabove, for some applications, antenna 28 is used to receive electrical power for powering the control circuitry 32 of control capsule 30 to drive a current via electrodes 22. Typically, the antenna receives power via inductive coupling, e.g., by transmitter 26 (shown in Fig. 1), or a different transmitter, transmitting RF energy toward antenna 28, such as to generate a magnetic field through the antenna. The magnetic field passing through antenna 28 generates an inductive current through antenna 28. The current through antenna 28 in turn generates a magnetic field, which can generate an inductive current through the body of stent 20, which may interfere with the antenna current, and reduce the efficiency of the electrical powering of the control circuitry of the control capsule. The antenna posts are configured such that, when the antenna is sutured to the antenna posts, the antenna is separated from the distal end of the stent body. For some applications, by separating the antenna from the distal end of the stent body, the posts reduce the strength of the inductive current that is generated in the stent body, thereby increasing the efficiency of the electrical powering of the control circuitry of the control capsule, via the inductive current that is generated through the antenna. For some applications, a length L (Fig. 3) of each of antenna posts 46 is less than 20 mm, e.g., less than 15 mm, and/or greater than 1 mm, e.g., greater than 5 mm.

As described hereinabove, stent 20 defines electrode posts 42, which are separate from antenna posts 46. The electrode posts and the antenna posts are configured such as to provide a longitudinal separation between the electrodes and the antenna. In this manner, electrical interference between the antenna and the electrodes is reduced relative to if, for example, the electrodes were to be placed upon the antenna posts.

Typically, antenna 28 is wiredly coupled to control circuitry 32 of control capsule 30 (wires shown in Fig. 3C), and the control circuitry of the control capsule is powered using the inductive current of the antenna. For some applications, the inductive current of the antenna is the only source of power for the control circuitry of the control capsule. The control circuitry of the control capsule is typically configured to drive a current into the blood vessel via electrode 22 (e.g., to stimulate the blood vessel), and/or to receive an electrical parameter of the blood vessel via the electrode. Typically, the control circuitry of the control capsule is wiredly coupled to electrode 22 (wires not shown), and, in cases in which there is more than one electrode 22, the control circuitry of the control capsule is wiredly coupled to each of electrodes 22. For some applications, stent 20 is shaped to define a wire holder 48 that is configured to hold in place, with respect to the stent body, the wires that couple the antenna and the electrode(s) to the control circuitry of the control capsule, by the wires being threaded through slots defined by the wire holder.

Reference is now made to Fig. 3A, which is a schematic illustration showing the structure of stent 20, in accordance with some applications of the present disclosure. Fig. 3A shows a flattened profile of the stent, which depicts (for illustrative purposes) how the stent would appear if a longitudinal incision were to be made along the length of the stent at a given circumferential location of the stent, and the stent were to then be laid out flat upon a surface. As shown in the enlarged portion on the top left of Fig. 3A, for some applications, one or more of antenna posts 46 include one or more flat portions 130 that protrude widthwise from a straight portion of the antenna post (e.g., straight distal portion 84, as shown in Fig. 3B). As described in further detail hereinbelow, with reference to Fig. 3B, typically antenna 28 is disposed annularly on the antenna posts, and a fastening element couples the antenna to the antenna posts by fastening the antenna against the one or more flat portions.

As shown in the enlarged portion on the top right of Fig. 3A, for some applications, one or more of the struts of ring 66 of struts (which is in the vicinity of wire holder 48) of stent 20 includes a widened, flat surface that is wider than most of the struts belonging to ring 66 of struts. Typically, the widened struts are disposed between electrode posts 42 and the location at which the control capsule is coupled to stent 20 (e.g., between electrode posts 42 and wire holder 48). For some applications, two or more of the struts are wider than most of the struts belonging to ring 66, and each of the two or more struts defines a widened, flat surface having a given width that is greater than the width of most of the struts belonging to ring 66. For some applications, the widths of the widened, flat surfaces defined by at least some of the wider struts differ from each other. For example, as shown in Fig. 3A, the widths of widened, flat surfaces 132 of two of the wider struts 154 and 156 is less than the width of widened flat surfaces 134 of two of the other wider struts 160 and 162. For some applications, the widened flat surfaces of the struts facilitate fastening (e.g., suturing) to stent 20 of the wires that couple control circuitry 32 of control capsule 30 to the electrodes, as described in further detail hereinbelow, with reference to Fig. 3C.

For some applications, at least one of struts of ring 67 of struts (which is the ring of struts that is closest to antenna posts 46) includes a widened flat surface that is wider than most of struts of strut ring 67. For example, as shown in Fig. 3A, strut 168 is wider than most of the struts of strut ring 66, strut 168 defining a widened flat surface 138 having a given width. For some applications, a second strut 170 of strut ring 67 is wider than most of the struts of strut ring 67, strut 170 defining a widened flat surface 140 having a given width that is greater than the width of flat surface 138. For some applications, the widened flat surfaces facilitate suturing to stent 20 of a wire that couples control circuitry 32 of control capsule 30 to the antenna 28 and/or the wire(s) that couple the control circuitry to the electrodes, as described in further detail hereinbelow, with reference to Fig. 3C.

Reference is now made to Fig. 3B, which is a schematic illustration of stent 20, with antenna 28 coupled thereto, in accordance with some applications of the present disclosure. Fig. 3B shows a flattened profile of the stent, which depicts (for illustrative purposes) how the stent would appear if a longitudinal incision were to be made along the length of the stent at a given circumferential location of the stent, and the stent were to then be laid out flat upon a surface. As described hereinabove, for some applications, one or more of antenna posts 46 includes one or more flat portions 130 that protrude widthwise from a straight portion of the antenna post (e.g., straight distal portion 84, as shown). Typically, each of the flat portions defines a flat surface having an area of at least 0.24 mm2. For some applications, a width W1 of each of the flat portions is greater than 0.4 mm (e.g., greater than 0.45 mm), and/or less than 0.7 mm (e.g., less than 0.6 mm), e.g., 0.4mm - 0.7 mm, or 0.45 mm - 0.6 mm. For some applications, a length L0 of each of the flat portions is greater than 0.6 mm (e.g., greater than 0.7 mm), and/or less than 1 mm (e.g., less than 0.9 mm), e.g., 0.6 mm - 1 mm, or 0.7 mm - 0.9 mm. Further typically, a ratio of the width W1 of each of the flat portions relative to a width W2 of the remainder of the straight portion of the electrode post is greater than 3:2, and/or less than 5:2, e.g., 3:2 - 5:2. As shown in Fig. 2B, for example, typically antenna 28 is disposed annularly on the antenna posts. For some applications, a plurality of sutures 141 couple the antenna to the antenna posts by fastening the antenna against the one or more flat portions of the antenna posts. For some applications, a different fastening element is used to fasten the antenna against the one or more flat portions. For example, glue, a hook and loop fastener (such as Velcro®), a tie, a pin, a staple, and/or a different fastening element may be used. Typically, by fastening the antenna against the one or more flat portions of the antenna posts, damage to the antenna is reduced relative to if the antenna were to be fastened against one of the edges of the antenna posts.

Reference is now made to Fig. 3C, which is a schematic illustration of stent 20, with electrodes 22 disposed on electrode posts 42 of the stent, in accordance with some applications of the present disclosure. Fig. 3C shows a flattened profile of the stent, which depicts (for illustrative purposes) how the stent would appear if a longitudinal incision were to be made along the length of the stent at a given circumferential location of the stent, and the stent were to then be laid out flat upon a surface. Typically, each of the electrodes is coupled, via a wire, to control circuitry 32 that is disposed in control capsule 30 (Fig. 2B). For some applications, the wires that couple the electrodes to the control circuitry of the control capsule are fastened against inner surfaces of struts of the stent (e.g., using sutures, and/or using a different type of the fastening elements described hereinabove). For example, the left-most electrode shown in Fig. 3C is coupled to the control circuitry via electrode wire 150, and the adjacent electrode to the left-most electrode is coupled to the control circuitry via electrode wire 152.

As described hereinabove, and as shown in Fig. 3A, for some applications, one or more of the struts of strut ring 66 of stent 20 includes a widened, flat surface that is wider than most of the struts belonging to strut ring 66. Typically, the widened struts are disposed between electrode posts 42 and the location at which the control capsule is coupled to stent 20 (e.g., between electrode posts 42 and wire holder 48). For some applications, the widened struts facilitate fastening (e.g., suturing) to stent 20 of the wires that couple the electrodes to control circuitry 32 of control capsule 30. For example, the portion of wire 150 that is closest to the left-most electrode is fastened against the inner surface of a strut that is not widened with respect to most of the struts of strut ring 66. Strut 154 of the stent, at which wire 152 begins to run alongside wire 150, is wider than most of the struts of strut ring 66, the strut defining widened flat surface 132 (shown in Fig. 3A). Similarly, strut 156, which is adjacent to strut 154, and upon which wire 152 continues to run alongside wire 150, defines widened flat surface 132 (which is generally similar to that of strut 154).

Typically, the width of most of the struts belonging to strut ring 66 is more than 150 micrometers, and/or less than 300 micrometers, e.g., 150-300 micrometers. Typically, the width of flat surface 132 is at least 300 micrometers (e.g. greater than 350 micrometers), and the diameter of each of electrode wires 150 and 152 is less than 150 micrometers. Thus, flat surface 132 is typically able to accommodate both wire 150 and wire 152 being fastened, alongside one another, directly against surface 132. By fastening the wires to the inner surface of the stent alongside one another, the radial diameter to which the stent may be constrained during insertion may be reduced relative to if, for example, wire 150 were to be fastened directly against the inner surface of the stent, and wire 152 were to be placed over wire 150 and fastened to wire 150, such that wire 150 separates between wire 152 and the inner surface of the stent. Furthermore, by accommodating wires 150 and 152 being fastened, alongside one another, directly against surface 132, surface 132 is configured to reduce the likelihood of either of the wires being squeezed between a gap between two of the stent struts, when the stent is radially constrained during insertion of the stent, relative to if surface 132 were unable to accommodate the two wires in the aforementioned manner.

For some applications, a third electrode wire 158 couples a third electrode to the control circuitry of the control capsule. As described hereinabove, struts 160 and 162 are wider than most of the struts belonging to strut ring 66, and define flat surfaces 134, that each have a width that is greater than the width of each of flat surfaces 132. Typically, the width of flat surface 134 is at least 400 micrometers (e.g., greater than 450 micrometers), and the diameter of each of electrode wires 150, 152 and 158 is less than 150 micrometers. (It is noted that the wires are not drawn to scale in Fig. 3C.) Flat surfaces 134 are typically each able to accommodate all of wires 150, 152, and 158 being fastened, alongside one another, directly against surface 134. As described hereinabove with reference to flat surfaces 132, typically by accommodating the electrode wires in the aforementioned manner, flat surfaces 134 (a) reduce the radial diameter to which the stent may be constrained during insertion, and/or (b) reduce the likelihood of the electrode wires being squeezed between struts of the stent, relative to of flat surfaces 134 were not to accommodate the electrode wires in the aforementioned manner. For some applications, flat surfaces 134 do not accommodate all of the electrode wires 150, 152 and 158 being fastened, alongside one another, directly against the surface. Even in such applications, the widths of the surfaces are typically such that the surfaces are able to accommodate at least two of the electrode wires being fastened alongside one another, directly against the surface.

For some applications, antenna 28 is coupled to control circuitry of the control capsule via an antenna wire 166. As described hereinabove, and as shown in Fig. 3A, for some applications, one or more of the struts of distal strut ring 67 includes a widened flat portion that is wider than most of the struts of strut ring 67. For example, as shown in Fig. 3A, strut 168 is wider than most of the struts of strut ring 67, strut 168 defining a widened flat surface 138 having a given width. For some applications, most of the struts of strut ring 67 have a width that is more than 200 micrometers, and/or less than 300 micrometers, e.g., 200-300 micrometers. Typically, the widened flat surface is sized such as to accommodate antenna wire 166. For example, a width of surface 138 may be at least 400 micrometers (e.g., greater than 450 micrometers), and the diameter of the antenna wire may be less than 400 micrometers. For some applications, the antenna wire, which couples the antenna to the control circuitry of the control capsule is fastened against widened flat surface 138 (e.g., using sutures, and/or using a different type of the fastening elements described hereinabove).

As described with reference to Fig. 3A, for some applications, a second strut 170 of strut ring 67 is wider than most of the struts of strut ring 67, strut 170 defining a widened flat surface 140 having a width that is greater than the width of flat surface 138. For some applications, widened, flat surface 140 is sized such as to accommodate antenna wire 166 and one or more electrode wires being fastened, alongside one another, directly against surface 140. As described hereinabove with reference to flat surfaces 132, typically by accommodating the antenna wire and electrode wire(s) in the aforementioned manner, flat surface 140 (a) reduces the radial diameter to which the stent may be constrained during insertion, and/or (b) reduces the likelihood of the antenna wire and/or the electrode wire(s) being squeezed between struts of the stent, relative to if flat surface 140 was not to accommodate the antenna wire and the electrode wire(s) in the aforementioned manner.

As described hereinabove, typically first, second, and third electrodes are coupled to control circuitry of the control capsule via electrode wires 150, 152 and 158, respectively. For some applications, a fourth electrode is coupled to the control circuitry via a fourth electrode wire 172, the fourth electrode wire typically having the same diameter as wire 150, 152, and 158. Typically, widened flat surface 140 accommodates antenna wire in addition to at least one of (e.g., between one and four of) electrode wires 150, 152, 158 and 172 being fastened, alongside one another, directly against surface 140. Typically, the width of widened flat surface 140 is at least 500 micrometers (e.g., more than 550 micrometers). Further typically, the diameter of each of electrode wires 150, 152, 158, and 172 is less than 150 micrometers mm, and the diameter of antenna wire is less than 400 micrometers.

Referring again to Fig. 3A, as described hereinabove, typically, stent 20 defines coupling elements 31 at a proximal end of the stent. During insertion of the stent via the delivery system, the delivery system holds the stent in place with respect to the catheter of the delivery system using the coupling elements. In order to place the stent inside the blood vessel at a deployment location, the catheter is retracted at the deployment location, such that the stent is released from the catheter. The stent becomes anchored to the blood vessel via radial expansion of the stent against the inner wall of the blood vessel. Subsequently, the coupling elements are decoupled from the delivery system, and the catheter is withdrawn from the blood vessel. For some applications, the handle of the delivery system (not shown) that is controlled by the physician is configured to display the rotational disposition of stent 20 to the physician. Typically, in order for the handle of the delivery system to correctly display the rotational disposition of the stent, the stent must be loaded onto the delivery system in a given rotational orientation with respect to the delivery system. Therefore, for some applications, one of coupling elements 31 has a different shape from the other coupling elements. For example, as shown in Fig. 3A, one of the coupling elements defines a circular opening, whereas the other coupling elements define square openings. The physician couples the stent to the delivery system by rotationally aligning the coupling element having the different shape from the other coupling elements (e.g., the circular coupling element in Fig. 3A) with a given portion of the delivery system.

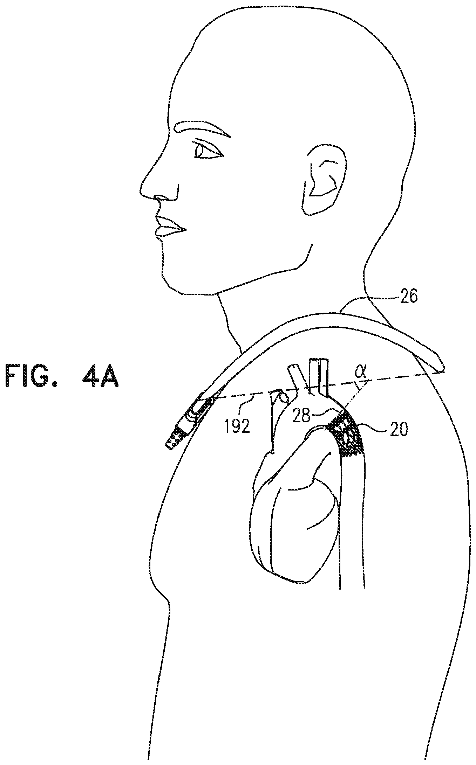

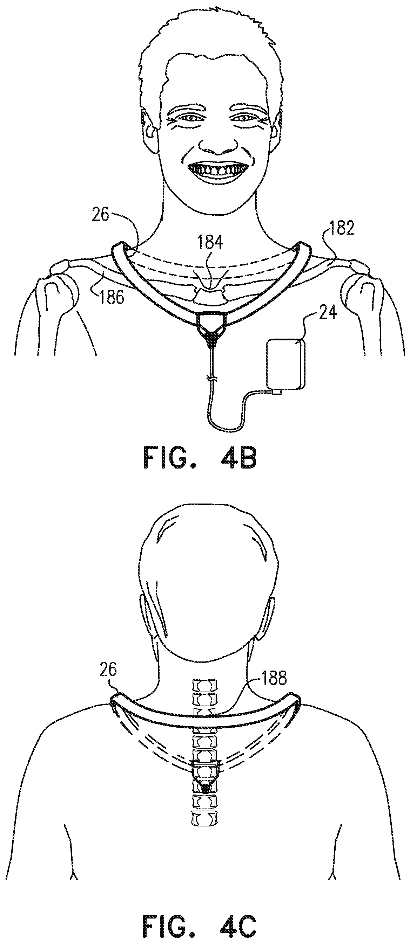

Reference is now made to Figs. 4A-C, which are schematic illustrations of respective views of transmitting antenna 26 disposed upon a subject's upper body, in accordance with some applications of the present disclosure. Figs, 4A shows a cross-sectional view of antenna 26 disposed on the subject's upper body, Fig. 4B shows a view of the front of the subject, and Fig. 4C shows a view of the subject's back. Typically, antenna 26 is configured to function as described hereinabove, with reference to Fig. 1. For some applications, control unit 24 transmits a signal and/or power toward stent 20, via antenna 26. In this manner, antenna 28 acts as a receiver and antenna 26 acts as a transmitter. Antenna 28 receives the transmitted signal and/or power, and control circuitry 32 of control capsule 30 drives the electrodes to drive a current into the blood vessel, in response to the antenna receiving the signal. For some applications, control circuitry 32 of control capsule 30 transmits data to control unit 24 by transmitting a signal from antenna 28 toward antenna 26. Thus, for some applications, antenna 28 acts as a transmitter and antenna 26 acts as a receiver. Typically, antenna 26 and antenna 28 transmit electrical energy between one another via electromagnetic inductive coupling. Although antenna 26 is shown disposed outside the subject's body, for some applications, antenna 26 is subcutaneously implanted within the subject's upper body.

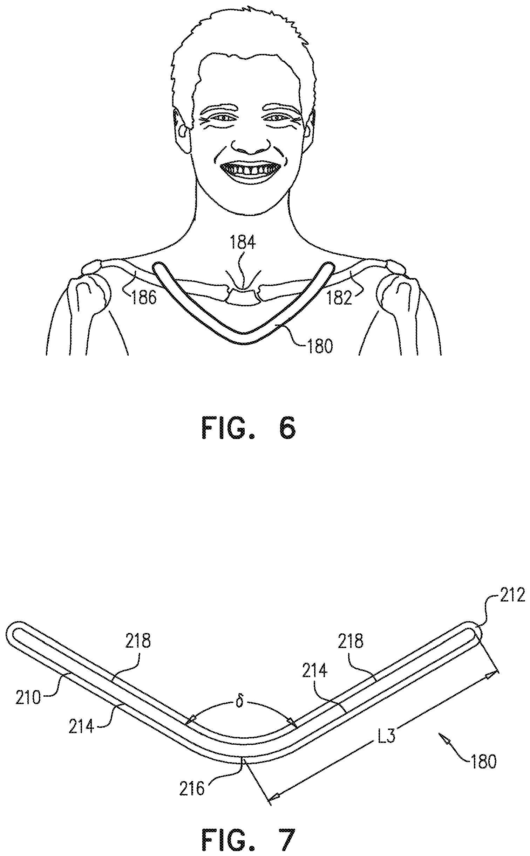

Antenna 26 is typically placed with respect to the subject (e.g., subcutaneously implanted within the subject, or placed outside the subject's skin) such that the antenna extends at least from above a left clavicle 182 of the subject to below a jugular notch 184 of the subject and from below the subject's jugular notch to above a right clavicle 186 of the subject. For some applications, antenna 26 circumscribes the subject's neck, as shown in Figs. 4A-C, a coil of the antenna (i.e., a turn of wire of the antenna) forming a complete loop that extends from below the subject's jugular notch to behind a vertebra 188 of the subject (e.g., behind a vertebra that is between the C1 and the T3 vertebrae, e.g., behind the T1 vertebra). Alternatively, antenna 26 does not extend such that the antenna circumscribes the subject's neck, as described hereinbelow with reference to Figs. 6 and 7.

A computer simulation was conducted by the inventors of the present application. Data were collected from CT images of 30 heart failure patients, of whom 28 patients had a CT resolution sufficient for data analysis. The heights and body-mass indices of each of the patients were recorded. CT measurements from the 28 heart failure patients were analyzed to determine, for each of the patients, if a first antenna were to be placed in the patient's aorta in the manner described herein with respect to antenna 28, and a second antenna were to be placed on the subject's upper body, in the manner described herein with reference to antenna 26, what would be (a) the height offset between the wire coils of the two antennas, (b) what would be the effective sagittal angle between the wire coils of the two antennas, and (c) what would be the effective transverse angle between the wire coils of the two antennas. In addition, for each of the patients, a coupling coefficient between the two antennas was measured using a geometrical phantom and using a Virtual Network Analyzer (VNA, Omicron BODE 100).

Typically, a wire coil through which an electrical current is driven generates magnetic flux lines that extend axially through the center of the coil perpendicularly to a plane defined by a 2D projection of the coil. As used herein, the effective sagittal angle is the angle, in the sagittal plane, between the planes defined by the effective 2D projections of antennas, perpendicular to which the magnetic flux lines generated by the respective antennas extend axially through the antennas. The plane defined by the effective 2D projection of antenna 26, perpendicular to which the magnetic flux lines generated by antenna 26 is indicated by dashed line 192, in Fig. 4A. The effective sagittal angle between antenna 28 and antenna 26 is indicated by angle *alpha* in Fig. 4A.

As stated hereinabove, for each of the patients, a coupling coefficient between the two antennas was measured. The coupling coefficient was determined using antennas having the following characteristics:

Aortic antenna (corresponding to antenna 28): A gold coil having 6 turns, the coil defining a diameter of 26 mm, and having an inductance of 1.75 micro-henries, and being disposed at an offset of 40 degrees from the horizontal in the sagittal plane.

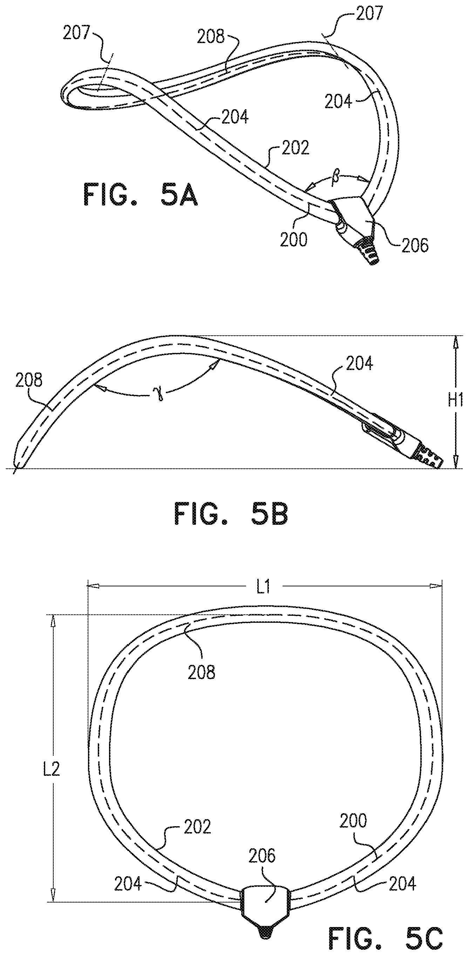

Upper body antenna (corresponding to antenna 26): A wire coil made of 3 turns of 26 AWG copper wire, the coil having an inductance of 6.85 micro-henries, and defining a major axis of 22 cm, and a minor axis of 19 cm (the major and minor axes being as defined hereinbelow with reference to Fig. 5C).

The coupling coefficient was defined as:

$$\text{coupling coefficient} = (V_{28}/V_{26}) * \sqrt{(L_{26}/L_{28})}$$

where $V_{28}$ and $V_{26}$ are the voltage gains of antenna 28 and 26, respectively, and $L_{28}$ and $L_{26}$ are the inductances of antenna 28 and 26, respectively.

In general, the inventors have found that it is typically the case that, in order for antennas 28 and 26 to communicate effectively through tissue of the subject's upper body, a coupling coefficient of greater than 0.004 is desirable. In the above described experiment, it was found that for the 28 patients, the mean coupling coefficient was 0.0109, with a standard deviation of 0.00136. The maximum coupling coefficient was 0.01515, and the minimum was 0.00857.

In addition to determining the coupling coefficients of the antennas for each of the patients, the correlation between the coupling coefficient and a number of parameters was determined. There was a negative correlation between the effective sagittal angular offset between the two antennas, and the coupling coefficient (i.e., as the angle *alpha* increased, the coupling coefficient decreased). Similarly, there was a negative correlation between the height offset between the two antennas, and the coupling coefficient (i.e., as the angle *alpha* increased, the coupling coefficient decreased). There was determined to be substantially no correlation between the coupling coefficients of the antennas and any of the following factors: (a) the patients' heights, (b) the patients' body-mass indices, and (c) the effective transverse angle between the coils of the two antennas.

As described hereinabove, typically antenna 28 and antenna 26 are configured to transmit electrical energy between one another via inductive coupling. In general, when a first and a second coil communicate via induction, other factors being equal, the coupling coefficient between first and second coils is maximized when the magnetic flux lines that are generated by the first coil are aligned with the magnetic flux lines defined by the second coil. Typically, this is the case when the effective angle between the two coils is zero. The coupling coefficient between first and second coils is zero when the magnetic flux lines that are generated by the first coil are at 90 degrees with respect to with the magnetic flux lines defined by the second coil. By placing antenna 26 upon the subject in the above-described manner, the effective sagittal angle *alpha* between antenna 26 and antenna 28 is typically between 20 and 70 degrees, e.g., between 30 and 60 degrees. Based upon the above described computer simulation that was conducted by the inventors of the present application, when antenna 28 and antenna 26 are disposed with respect to one another in this configuration, the coupling coefficient between the two antennas is typically at least greater than 0.004, and is typically greater than 0.008. Therefore, the antennas are able to transfer electrical energy via inductive coupling, when the antennas are disposed with respect to one another in the configurations described herein.

Reference is now made to Figs. 5A-C, which are schematic illustrations of respective views of antenna 26, in accordance with some applications of the present disclosure. Typically, antenna 26 includes a coil 200 disposed inside a housing 202. As shown, typically the antenna (e.g., the housing and/or coils of the antenna) typically defines a saddle shape. The coil wire defines first and second elongate portions 204 that diverge from a bifurcation 206 at a first end of the first elongate portion and a first end of the second elongate portion to a second end of the first elongate portion and a second end of the second elongate portion. Typically, the elongate portions diverge from one another at an angle *beta* that is greater than 80 degrees (e.g., greater than 90 degrees), and/or less than 130 degrees (e.g., less than 120 degrees), e.g., between 80 and 130 degrees, or between 90 and 120 degrees. The coil also includes a curved portion 208 that extends from the second end of the first elongate portion to the second end of the second elongate portion. Although the wire is shown defining a single complete turn, for some applications, the coil defines a plurality of turns. For some applications, the coil defines more than three turns and/or less than eight turns (e.g., less than six turns). For example, the coil may define 2-8, e.g., 3-6 turns. In accordance with respective applications, when the coil defines a plurality of turns, the turns of the coil are continuous with each other, or the turns are separate from one another, each of the turns defining a separate closed loop.

Typically, the length of each of elongate portions 204 (i.e., the length measured along coil 200 from dashed line 207 to bifurcation 206) is greater than 15 cm (e.g., greater than 20 cm), and/or less than 40 cm (e.g., less than 30 cm), e.g., 15-40 cm, or 20-30 cm. Further typically, the length of curved portion 208 (i.e., the length measured along the along the wire from the first of dashed lines 207 to the second of dashed lines 207) is greater than 15 cm (e.g., greater than 20 cm), and/or less than 40 cm (e.g., less than 30 cm), e.g., 15-40 cm, or 20-30 cm.

With reference to Fig. 5B, coil 200 is typically shaped such that the wire has an L-shaped projection in the sagittal plane. An angle *gamma* between the two legs of the L-shaped projection is typically greater than 80 degrees (e.g., greater than 100 degrees), and/or less than 160 degrees (e.g., less than 140 degrees), e.g., between 80 and 160 degrees, or between 100 and 140 degrees. The L-shape is typically such that, if the antenna is placed upon a flat surface, a height HI of antenna 26 is more than 2 cm (e.g., more than 3 cm), and/or less than 8 cm (e.g., less than 7 cm), e.g., 2-8 cm, or 3-7 cm. With reference to Fig. 5C, coil 200 is typically shaped such that the coil has a pear-shaped projection in the transverse plane. A length L1 of a first axis of the pear-shape defined by coil 200 is typically more than 8 cm (e.g., more than 10 cm), and/or less than 22 cm (e.g., less than 20 cm), e.g., 8-22 cm, or 10-20 cm. A length L2 of a second axis of the pear-shape defined by coil 200 is typically more than 12 cm (e.g., more than 15 cm), and/or less than 30 cm (e.g., less than 25 cm), e.g., 12-20 cm, or 15-25 cm.

Although Figs. 5A-C show coil 200 disposed inside a housing that has the same general shape as the coil, the scope of the present application includes using a different type of housing to house the coil. For example, the coil may be housed inside a vest (i.e., an undershirt), inside a bra-like housing, inside tacky silicone that sticks to the subject's skin, or may be supported upon the subject's upper body using strings. As described hereinabove, for some applications, antenna 26 (e.g., coil 200 of antenna 26) is implanted subcutaneously.

Reference is now made to Fig. 6, which is a schematic illustration of antenna 26 placed on a subject's upper body, in accordance with some applications of the present disclosure. Antenna 26 as shown in Fig. 6 is generally similar to antenna 26 shown in Figs. 4A-C and 5A-C, except that antenna 26 as shown in Fig. 6 is not configured to circumscribe the subject's neck. Rather, the antenna is configured to be placed on the subject's chest such that the antenna extends at least from above the subject's left clavicle 182 to below the subject's jugular notch 184 and from below the subject's jugular notch to above the subject's right clavicle 186. As described with reference to Figs. 4A-C, antenna 26 is configured to transmit electrical data and power to antenna 28, and, for some applications, is additionally configured to receive electrical data from antenna 28.

Fig. 7 is a schematic illustration of antenna 26, as shown in Fig. 6, in accordance with some applications of the present disclosure. For some applications, antenna 26 includes a coil 210 disposed inside a housing 212. The coil defines first and second elongate portions 214 that diverge from a bifurcation 216 at a first end of the first elongate portion and a first end of the second elongate portion to a second end of the first elongate portion and a second end of the second elongate portion. Typically, the elongate portions diverge from one another at an angle *delta* that is greater than 80 degrees (e.g., greater than 90 degrees), and/or less than 130 degrees (e.g., less than 120 degrees), e.g., between 80 and 130 degrees, or between 90 and 120 degrees. Angle *delta* is typically the same as angle *beta* described hereinabove. The coil also includes third and fourth elongate portions 218 that are parallel, respectively, to the first and second elongate portions, and that extend from the second ends of the first and second elongate portions back toward bifurcation 216. The first, second, third and fourth elongate portions comprise portions of a single closed loop. Although the coil is shown defining a single turn, for some applications, the coil defines a plurality of turns. For some applications, the coil defines more than three turns and/or less than eight turns (e.g., less than six turns). For example, the coil may define 2-8, e.g., 3-6 turns. In accordance with respective applications, when the coil defines a plurality of turns, the turns of the coil are continuous with each other, or the turns are separate from one another, each of the turns defining a separate closed loop.

Typically, a length L3 of each of elongate portions 214 (i.e., the length measured along coil 210 from where the coil loops until bifurcation 216) is greater than 15 cm (e.g., greater than 20 cm), and/or less than 40 cm (e.g., less than 30 cm), e.g., 15-40 cm, or 20-30 cm.

As described hereinabove, with reference to Figs. 5A-C, although Fig. 7 shows coil 210 disposed inside a housing that has the same general shape as the coil, the scope of the present application includes using a different type of housing to house the coil. For example, the coil may be housed inside a vest (i.e., an undershirt), inside a bra-like housing, inside tacky silicone that sticks to the subject's skin, or may be supported upon the subject's upper body using strings. As described hereinabove, for some applications, antenna 26 (e.g., coil 210 of antenna 26) is implanted subcutaneously.

Reference is now made to Fig. 8, which is a schematic illustration of a portion of antenna 26, in accordance with some applications of the present disclosure. Fig. 8 shows a view of the antenna in which a top portion 202A of housing 202 of the antenna has been separated from a bottom portion 202B of the housing, such that coil 200 of the antenna is exposed. In the application shown in Fig. 8, the antenna coil has three turns of wire 200A, 200B and 200C, and the turns are separate from one another, each of the turns defining a separate closed loop. For some applications, each of the turns of the coil is electrically coupled to a printed circuit board ("PCB") 220 that is disposed inside the housing. For some applications, antenna 26 is configured to be wiredly coupled to control unit 24. For example, as shown, a wire 224 may extend from PCB 220 to control unit 24.

For some applications, each of the turns of coil 200 of antenna 26 is electrically coupled to a respective capacitor 222. For example, as shown in Fig. 8, there are three turns of the coil, and each of the turns is coupled to a capacitor. Capacitors 222 are configured to increase resilience of the antenna relative to if the antenna were not to include capacitors 222. For some applications, the capacitors are configured to prevent the antenna from becoming detuned. For example, the capacitors may prevent the antenna from becoming detuned as a result of becoming misshapen.

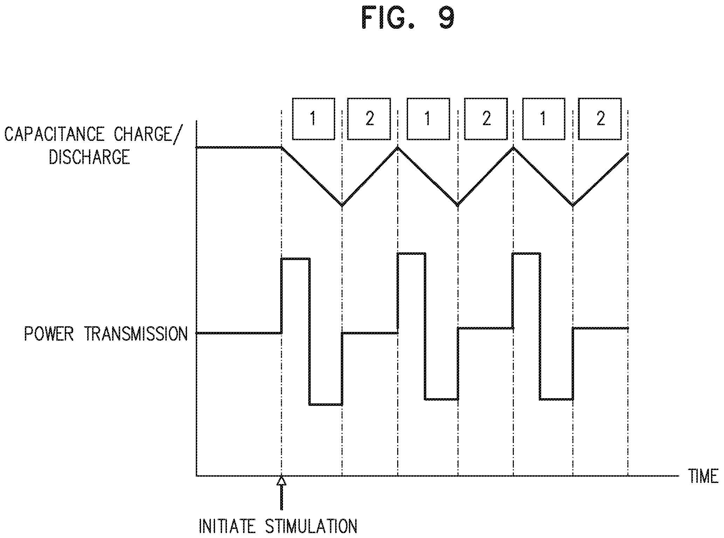

Reference is now made to Fig. 9, which is a graph showing (a) the charging and discharging cycle of capacitor 33 of control circuitry 32 of control capsule 30 of stent 20 (see Fig. 2B), and (b) the cycle of transmission of power from transmitter 26 (e.g., antenna 26) to antenna 28 of stent 20, in accordance with some applications of the present disclosure.

As shown schematically in Fig. 9, at the initiation of stimulation of the subject's blood vessel by the control circuitry 32, the capacitance of capacitor 33 of control circuitry 32 discharges, in order to drive a current into the blood vessel via electrode 22. During the discharging of the capacitor, power is transmitted from transmitting antenna 26 to antenna 28 of stent 20, as denoted by the bottom portion of the graph. This type of period, during which the capacitor discharges and power is transmitted from the transmitter to the antenna is denoted period type 1, as indicated by the box with the "1" inside at the top of the graph.

During a subsequent time period, the capacitor of the control circuitry charges, using the power that was transmitted to antenna 28 during the previous time period. During this time period, there is no transmission of power from transmitter 26 to antenna 28. This type of period, during which the capacitor charges, and power is not transmitted from the transmitter to the antenna, is denoted period type 2, as indicated by the box with the "2" inside at the top of the graph.

For some applications, for the duration of the stimulation of the blood vessel by control circuitry 32, the charge-discharge cycle of the capacitor, and the cycle of transmission of power from transmitter 26 to antenna 28 alternates between period types 1 and 2, as illustrated in Fig. 9. In this manner, power is repeatedly transmitted to antenna 28, while the capacitor discharges, such that the power is available for charging of the capacitor as soon as the discharging of the capacitor terminates. For some applications, by performing capacitor charging/discharging and power transmission in accordance with the above-described cycle, the transmitter is able to transmit power to the antenna in real time with respect to the stimulation of the blood vessel by the control circuitry. Thus, the control circuitry is not required to store all of the electrical energy that will be required for the stimulation of the blood vessel in advance of the initiation of the stimulation of the blood vessel.

For some applications, the techniques described with reference to Fig. 9 are used to operate any combination of one or more electrodes, a capacitor and a first antenna that are electrically coupled to each other, and that are disposed inside a subject's body.

It is noted that in the context of the present disclosure, the term coil is used to denote a conductive wire that defines one or more turns that form one or more complete closed loops. The term coil should not be interpreted to be limited to any particular shape.

It is noted that, although some applications of the present disclosure have been described as being used in conjunction with a stent, the scope of the present disclosure includes applying the apparatus and methods described herein to a stent graft, *mutatis mutandis.* For example, an antenna may be coupled to the body of a stent graft via posts that longitudinally separate the antenna from a distal end of the body of the stent graft, in accordance with the techniques described hereinabove. It is further noted that the term "stent" as used in the present disclosure should not be interpreted as being limited to a device that is configured to hold open a bodily lumen. Rather the term "stent" as used herein should be interpreted as being interchangeable with the term "scaffold," the term denoting a device that is configured to support elements inside a bodily lumen, e.g., a blood vessel, such as the aorta.

It is noted that although some applications of the present disclosure have been described as being used in conjunction with a stent that is placed inside a subject's aorta, the scope of the present disclosure includes applying the apparatus and methods described herein to a stent that is placed inside a different blood vessel, *mutatis mutandis.* For example, a stent as described herein may be placed in the abdominal aorta, the vena cava, veins of the leg, the pulmonary artery, the pulmonary vein, the jugular vein, a carotid artery, the subclavian artery, a hepatic vein, a hepatic artery, a renal vein, a renal artery, a femoral vein, a femoral artery, and/or a different blood vessel of a subject, and techniques as described herein may be practiced using such a stent. Similarly, although the stent is shown at least partially disposed in the descending aorta, the scope of the present disclosure includes placing the stent at any location within the aorta, such as in the ascending aorta, the descending aorta, the aortic arch, or a combination thereof.

For example, the stent may be used to stimulate a neural pathway by driving a current into the wall of the blood vessel in which the stent is implanted, in accordance with the techniques described herein. Alternatively or additionally, the stent may be placed in the renal artery, in order to treat renal dysfunction, and/or in the pulmonary artery, in order to treat pulmonary hypertension. Further alternatively or additionally, the stent may be placed in the pulmonary artery and/or the carotid artery in order to be used for vagal stimulation (e.g., vasovagal stimulation), for example, in order to treat gastroesophageal reflux disease (GERD).

For some applications, the subject's cardiac cycle is determined by detecting an electrical signal from the subject's aorta, via electrodes 22, and deriving the subject's ECG and/or blood pressure from the electrical signal detected at the aorta, e.g., in accordance with techniques described in US 12/792,227 to Gross (published as US 2010/0305392). For some applications, physiological parameters of the subject (such as the subject's cardiac cycle) are detecting using antenna 28, for example using techniques as described in US 2014/0180391 For some applications, electrical stimulation is applied to the aorta in coordination with the subject's cardiac cycle, based upon the signal detected at the aorta.

For some applications, in response to detecting that a subject is undergoing an epileptic seizure, the subject's vagus nerve is stimulated by driving a current into the subject's aorta. For some applications, a current is driven into the subject's aorta in order to treat the subject for sleep apnea.

For some applications, the techniques described herein are practiced in combination with techniques described in any one of the following applications:

International Application PCT/IL2014/050972 to Dagan (published as WO 15/068167), filed November 06, 2014, entitled "Wireless endovascular stent-based electrodes," which claims priority from US Provisional Patent Application 61/900,461 to Dagan, entitled "Wireless endovascular stent-based electrodes," filed November 06, 2013;

International Application PCT/IL2013/050375 (published as WO 13/164829), filed May 02, 2013, entitled "Wireless endovascular stent-based electrodes," which claims priority from the following US provisional patent applications:

- US Provisional Patent Application 61/641,388 to Dagan, filed May 02, 2012, entitled "Wireless endovascular stent-based electrodes,"
- US Provisional Patent Application 61/714,277 to Dagan, filed October 16, 2012, entitled, "Wireless endovascular stent-based electrodes," and
- US Provisional Patent Application 61/773,919 to Dagan, filed March 07, 2013, entitled, "Wireless endovascular stent-based electrodes;"

US 13/741,154 to Dagan (published as 2014/0180391), which is the US national phase of International Application PCT/IL2012/000336 (published as WO 13/035092), filed September 09, 2012, entitled "Wireless endovascular stent-based electrodes," which claims priority from US Provisional Patent Application 61/532,660 to Dagan, filed September 09, 2011, entitled, "Wireless endovascular stent-based electrodes;"

US 13/210,778 to Dagan (issued as US 8,626,290), filed August 16, 2011, which is a continuation-in-part of US 12/957,799 to Gross (issued as US 8,626,299), filed December 01, 2010, entitled "Thoracic aorta and vagus nerve stimulation," which is a continuation-in-part of US 12/792,227 to Gross (published as US 2010/0305392), filed June 02, 2010, entitled "Thoracic aorta and vagus nerve stimulation," which claims the benefit of (a) US Provisional Patent Application 61/183,319 to Reisner, filed June 02, 2009, entitled "Thoracic aorta and vagus nerve stimulation," and (b) US Provisional Patent Application 61/331,453 to Dagan, filed May 05, 2010, entitled "Thoracic aorta and vagus nerve stimulation;"

US 12/023,896 to Gross (issued as US 9,005,106), filed January 31, 2008, entitled "Intra-aortic electrical counterpulsation;" and

US 11/995,904 to Gross (issued as US 8,862,243), which is the US national phase of International Application PCT/IL2006/000856 to Gross (published as WO 07/013065), filed July 25, 2006, entitled "Electrical stimulation of blood vessels," which claims priority from: (a) US Provisional Application 60/702,491, filed July 25, 2005, entitled, "Electrical stimulation of blood vessels," and (b) US Provisional Application 60/721,728, filed September 28, 2005, entitled, "Electrical stimulation of blood vessels."

It will be appreciated by persons skilled in the art that the present invention is not limited to what has been particularly shown and described hereinabove. Rather, the scope of the present invention includes variations and modifications without departing from the scope of the invention as defined by the appended claims.

**Claims**

1.  Apparatus comprising:

    a stent (20) configured to be placed inside an aorta of a subject, the stent (20) comprising one or more electrodes (22), control circuitry (32), and a first antenna (28) coupled thereto; and
    a second antenna (26) configured to be placed on the subject, the second antenna (26) being configured to transmit an electrical signal to the first antenna (28) via inductive coupling,
    **characterized in that**:

    the second antenna (26) is configured, when placed on the subject, to extend at least from above a left clavicle of the subject to below a jugular notch of the subject and from below the subject's jugular notch to above a right clavicle of the subject; and
    the second antenna (26) is shaped such that the antenna has an L-shaped projection in a sagittal plane of the subject, and an angle ($\gamma$) between two legs (204,208) of the L-shaped projection is 80-160 degrees when the second antenna (26) is placed on a flat surface.

2.  The apparatus according to claim 1 wherein the angle ($\gamma$) between the two legs (204,208) is greater than 100 degrees.

3.  The apparatus according to claim 1, wherein the second antenna (26) is shaped to define first and second elongate portions (204) that diverge from one another at an angle ($\beta$) that is greater than 80 degrees and less than 130 degrees.

4.  The apparatus according to claim 3, wherein the first and second elongate portions (204) diverge from one another at an angle ($\beta$) that is less than 120 degrees.

5.  The apparatus according to claim 1, wherein the second antenna (26) is configured to transmit power to the first antenna (28), and wherein the control circuitry (32) is configured to drive a current into the subject's aorta, via the electrodes (22), using the received power.

6.  The apparatus according to claim 1, wherein the stent (20) and the second antenna (26) are configured such that when the stent (20) is placed in the aorta, and the second antenna (26) is placed on the subject such that the second antenna (26) extends at least from above the subject's left clavicle to below the subject's jugular notch and from below the subject's jugular notch to above the subject's right clavicle, a coupling coefficient between the first and second antennas (28,26) is greater than 0.004, the coupling coefficient being defined as:

$$\text{coupling coefficient} = (V_1/V_2) * \sqrt{(L_2/L_1)}$$

    where $V_1$ and $V_2$ are voltage gains of the first and second antennas (28,26), respectively, and $L_1$ and $L_2$ are inductances of the first and second antennas (28,26), respectively.

7.  The apparatus according to claim 1, wherein the second antenna (26) is configured to be implanted subcutaneously.

8.  The apparatus according to claim 1, wherein the second antenna (26) is configured not to circumscribe a neck of the subject.

9.  The apparatus according to any one of claims 1, and 5-7, wherein the second antenna (26) is configured to circumscribe a neck of the subject.

10. The apparatus according to claim 9, wherein the second antenna (26) comprises at least one turn of wire configured to form a complete loop that extends from below the subject's jugular notch to behind a vertebra of the subject that is between C1 and T3 vertebrae.

11. The apparatus according to any one of claims 1, and 5-8, wherein the stent (20) and the second antenna (26) are configured such that when the stent (20) is placed in the aorta, and the second antenna (26) is placed on the subject such that the second antenna (26) extends at least from above the subject's left clavicle to below the subject's jugular notch and from below the subject's jugular notch to above the subject's right clavicle, an effective sagittal angle between the first and second antennas (28,26) is between 20 and 70 degrees.

12. The apparatus according to claim 11, wherein the stent (20) and the second antenna (26) are configured such that when the stent (20) is placed in the aorta, and the second antenna (26) is placed on the subject such that the second antenna (26) extends at least from above the subject's left clavicle to below the subject's jugular notch and from below the subject's jugular notch to above the subject's right clavicle, the effective sagittal angle between the first and second antennas (28,26) is between 30 and 60 degrees.

13. The apparatus according to any one of claims 1, and 5-8, wherein the second antenna (26) comprises a plurality of turns of wire, and a plurality of capacitors, and wherein each of the turns of wire is coupled to a respective one of the capacitors.

**Patentansprüche**

1. Vorrichtung, umfassend:

   einen Stent (20), der konfiguriert ist, um in einer Aorta eines Subjekts platziert zu werden, wobei der Stent (20) eine oder mehrere Elektroden (22), Steuerschaltungen (32) und eine damit gekoppelte erste Antenne (28) umfasst; und
   eine zweite Antenne (26), die konfiguriert ist, um am Subjekt platziert zu werden, wobei die zweite Antenne (26) konfiguriert ist, um durch induktive Kopplung ein elektrisches Signal an die erste Antenne (28) zu senden, **dadurch gekennzeichnet, dass**:

   die zweite Antenne (26) konfiguriert ist, wenn sie am Subjekt platziert ist, von mindestens oberhalb einer linken Clavicula des Subjekts bis unterhalb einer Jugularvenenengstelle des Subjekts und von unterhalb der Jugularvenenengstelle bis oberhalb einer rechten Clavicula des Subjekts zu verlaufen; und
   die zweite Antenne (26) so geformt ist, dass die Antenne in einer Sagittalebene des Subjekts einen L-förmigen Vorsprung aufweist, und ein Winkel ($\gamma$) zwischen zwei Schenkeln (204, 208) des L-förmigen Vorsprungs 80 bis 160 Grad ist, wenn die zweite Antenne (26) auf einer ebenen Oberfläche platziert ist.

2. Vorrichtung nach Anspruch 1, wobei der Winkel ($\gamma$) zwischen den zwei Schenkeln (204, 208) größer als 100 Grad ist.

3. Vorrichtung nach Anspruch 1, wobei die zweite Antenne (26) geformt ist, um erste und zweite längliche Abschnitte (204) zu definieren, die um einen Winkel von ($\beta$), der größer als 80 Grad und kleiner als 130 Grad ist, voneinander divergieren.

4. Vorrichtung nach Anspruch 3, wobei die ersten und zweiten länglichen Abschnitte (204) um einen Winkel von ($\beta$), der kleiner als 120 Grad ist, voneinander divergieren.

5. Vorrichtung nach Anspruch 1, wobei die zweite Antenne (26) konfiguriert ist, um Leistung an die erste Antenne (28) zu übermitteln, und wobei die Steuerschaltungen (32) konfiguriert sind, um mithilfe der empfangenen Leistung einen Strom über die Elektroden (22) in die Aorta des Subjekts zu treiben.

6. Vorrichtung nach Anspruch 1, wobei der Stent (20) und die zweite Antenne (26) so konfiguriert sind, dass, wenn der Stent (20) in der Aorta platziert ist und die zweite Antenne (26) am Subjekt platziert ist, so dass die zweite Antenne (26) mindestens von oberhalb der linken Clavicula des Subjekts bis unterhalb der Jugularvenenengstelle des Subjekts und von unterhalb der Jugularvenenengstelle des Subjekts bis oberhalb der rechten Clavicula des Subjekts verläuft, ein Kopplungskoeffizient zwischen den ersten und zweiten Antennen (28, 26) größer als 0,004 ist, wobei der Kopplungskoeffizient definiert ist als:

$$\text{Kopplungskoeffizient} = (V_1/V_2) * \sqrt{(L_2/L_1)}$$

wobei $V_1$ und $V_2$ Spannungsverstärkungen der ersten bzw. zweiten Antennen (28, 26) sind und $L_1$ und $L_2$ Induktivitäten der ersten bzw. zweiten Antennen (28, 26) sind.

7. Vorrichtung nach Anspruch 1, wobei die zweite Antenne (26) konfiguriert ist, um subkutan implantiert zu werden.

8. Vorrichtung nach Anspruch 1, wobei die zweite Antenne (26) konfiguriert ist, um nicht einen Hals des Subjekts zu umgeben.

9. Vorrichtung nach einem der Ansprüche 1 und 5 bis 7, wobei die zweite Antenne (26) konfiguriert ist, um einen Hals des Subjekts zu umgeben.

10. Vorrichtung nach Anspruch 9, wobei die zweite Antenne (26) mindestens eine Wicklung aus Draht umfasst, die konfiguriert ist, um eine vollständige Schleife zu bilden, die von unterhalb der Jugularvenenengstelle des Subjekts bis hinter einen Wirbelkörper des Subjekts, der zwischen Wirbelkörper C1 und T3 ist, verläuft.

11. Vorrichtung nach einem der Ansprüche 1 und 5 bis 8, wobei der Stent (20) und die zweite Antenne (26) so konfiguriert sind, dass, wenn der Stent (20) in der Aorta platziert ist und die zweite Antenne (26) am Subjekt platziert ist, so dass die zweite Antenne (26) mindestens von oberhalb der linken Clavicula des Subjekts bis unterhalb der Jugularvenenengstelle des Subjekts und von unterhalb der Jugularvenenengstelle des Subjekts bis oberhalb der rechten Clavicula des Subjekts verläuft, ein effektiver Sagittalwinkel zwischen den ersten und zweiten Antennen (28, 26) zwischen 20 und 70 Grad ist.

12. Vorrichtung nach Anspruch 11, wobei der Stent (20) und die zweite Antenne (26) so konfiguriert sind, dass, wenn der Stent (20) in der Aorta platziert ist und die zweite Antenne (26) am Subjekt platziert ist, so dass die zweite Antenne (26) mindestens von oberhalb der linken Clavicula des Subjekts bis unterhalb der Jugularvenenengstelle des Subjekts und von unterhalb der Jugularvenenengstelle des Subjekts bis oberhalb der rechten Clavicula des Subjekts verläuft, der effektive Sagittalwinkel zwischen den ersten und zweiten Antennen (28, 26) zwischen 30 und 60 Grad ist.

13. Vorrichtung nach einem der Ansprüche 1 und 5 bis 8, wobei die zweite Antenne (26) eine Vielzahl von Wicklungen aus Draht und eine Vielzahl von Kondensatoren umfasst, und wobei jede der Wicklungen aus Draht mit einem jeweiligen der Kondensatoren gekoppelt ist.

**Revendications**

1. Appareil comprenant :

un stent (20) conçu pour être placé à l'intérieur de l'aorte d'un sujet, le stent (20) comprenant une ou plusieurs électrodes (22), un circuit de commande (32) et une première antenne (28) couplée à celui-ci ; et
une seconde antenne (26) conçue pour être placée sur le sujet, la seconde antenne (26) étant conçue pour transmettre un signal électrique à la première antenne (28) via un couplage inductif,
**caractérisé en ce que** :

la seconde antenne (26) est conçue, lorsqu'elle est placée sur le sujet, pour s'étendre au moins du dessus de la clavicule gauche du sujet jusqu'au dessous d'une encoche jugulaire du sujet et du dessous de l'encoche jugulaire du sujet jusqu'au dessus de la clavicule droite du sujet ; et
la seconde antenne (26) est façonnée de sorte que l'antenne comporte une partie saillante en forme de L dans un plan sagittal du sujet et l'angle (y) entre deux pattes (204, 208) de la partie saillante en forme de L fasse 80 à 160 degrés lorsque la seconde antenne (26) est placée sur une surface plane.

2. Appareil selon la revendication 1, ledit angle ($\gamma$) entre les deux pattes (204, 208) étant supérieur à 100 degrés.

3. Appareil selon la revendication 1, ladite seconde antenne (26) étant façonnée pour définir des première et seconde parties allongées (204) qui divergent l'une de l'autre d'un angle ($\beta$) qui est supérieur à 80 degrés et inférieur à 130 degrés.

4. Appareil selon la revendication 3, lesdites première et seconde parties allongées (204) divergeant l'une de l'autre d'un angle (β) qui est inférieur à 120 degrés.

5. Appareil selon la revendication 1, ladite seconde antenne (26) étant conçue pour transmettre de l'énergie à la première antenne (28) et ledit circuit de commande (32) étant conçu pour envoyer un courant dans l'aorte du sujet via les électrodes (22) à l'aide de l'énergie reçue.

6. Appareil selon la revendication 1, ledit stent (20) et ladite seconde antenne (26) étant conçus de sorte que lorsque le stent (20) est placé dans l'aorte et la seconde antenne (26) est placée sur le sujet de sorte que la seconde antenne (26) s'étende au moins du dessus de la clavicule gauche du sujet jusqu'au dessous de l'encoche jugulaire du sujet et du dessous de l'encoche jugulaire du sujet jusqu'au dessus de la clavicule droite du sujet, le coefficient de couplage entre la première et la seconde antenne (28,26) soit supérieur à 0,004, le coefficient de couplage étant défini comme étant :

$$\text{coefficient de couplage} = (V_1/V_2) * \sqrt{(L_2/L_1)}$$

ou $V_1$ et $V_2$ représentent les gains de tension des première et seconde antennes (28,26), respectivement, et $L_1$ et $L_2$ représentent les inductances des première et seconde antennes (28,26), respectivement.

7. Appareil selon la revendication 1, ladite seconde antenne (26) étant conçue pour être implantée par voie sous-cutanée.

8. Appareil selon la revendication 1, ladite seconde antenne (26) étant conçue pour ne pas entourer le cou du sujet.

9. Appareil selon l'une quelconque des revendications 1 et 5 à 7, ladite seconde antenne (26) étant conçue pour entourer le cou du sujet.

10. Appareil selon la revendication 9, ladite seconde antenne (26) comprenant au moins un tour de fil conçu pour former une boucle complète qui s'étend du dessous de l'encoche jugulaire du sujet à derrière une vertèbre du sujet qui se trouve entre les vertèbres C1 et T3.

11. Appareil selon l'une quelconque des revendications 1 et 5 à 8, ledit stent (20) et ladite seconde antenne (26) étant conçus de sorte que lorsque le stent (20) est placé dans l'aorte et la seconde antenne (26) est placée sur le sujet de sorte que la seconde antenne (26) s'étende au moins du dessus de la clavicule gauche du sujet jusqu'au dessous de l'encoche jugulaire du sujet et du dessous de l'encoche jugulaire du sujet jusqu'au dessus de la clavicule droite du sujet, l'angle sagittal effectif entre les première et seconde antennes (28,26) soit compris entre 20 et 70 degrés.

12. Appareil selon la revendication 11, ledit stent (20) et ladite seconde antenne (26) étant conçus de sorte que lorsque le stent (20) est placé dans l'aorte et la seconde antenne (26) est placée sur le sujet de sorte que la seconde antenne (26) s'étende au moins du dessus de la clavicule gauche du sujet jusqu'au dessous de l'encoche jugulaire du sujet et du dessous de l'encoche jugulaire du sujet jusqu'au dessus de la clavicule droite du sujet, l'angle sagittal effectif entre les première et seconde antennes (28,26) soit compris entre 30 et 60 degrés.

13. Appareil selon l'une quelconque des revendications 1 et 5 à 8, ladite seconde antenne (26) comprenant une pluralité de tours de fil et une pluralité de condensateurs et chacun des tours de fil étant couplé à l'un respectif des condensateurs.

FIG. 1

FIG. 2A

FIG. 2B

FIG. 3A

FIG. 3B

FIG. 3C

FIG. 4A

FIG. 4B

FIG. 4C

FIG. 5A

FIG. 5B

FIG. 5C

FIG. 6

FIG. 7

FIG. 8

# FIG. 9

CAPACITANCE CHARGE/
DISCHARGE

POWER TRANSMISSION

TIME

INITIATE STIMULATION

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 13210778 B, Dagan
- US 20120035679 A
- US 12957799 B, Gross
- US 20110137370 A
- US 12792227 B, Gross
- US 20100305392 A
- US 8626290 B
- US 8626299 B
- US 20140180391 A
- IL 2014050972 W, Dagan
- WO 15068167 A
- US 61900461
- IL 2013050375 W
- WO 13164829 A
- US 61641388, Dagan

- US 61714277, Dagan
- US 61773919, Dagan
- US 13741154 B, Dagan
- IL 2012000336 W
- WO 13035092 A
- US 61532660, Dagan
- US 61183319, Reisner
- US 61331453, Dagan
- US 12023896 B, Gross
- US 9005106 B
- US 11995904 B, Gross
- US 8862243 B
- IL 2006000856 W, Gross
- WO 07013065 A
- US 70249105
- US 72172805